# EUROPEAN PATENT APPLICATION

(11) **EP 4 501 972 A1**
(43) Date of publication of application: **05.02.2025**
(21) Application number: 23779039.9
(22) Date of filing: 20.02.2023
(51) Int. Cl.: C08F 2/44, A61K 6/76, A61K 6/871, A61K 6/887, C01B 33/145

(54) **METHOD FOR PRODUCING TREATED-SILICA DISPERSED MONOMER COMPOSITION**

(30) Priority: 31.03.2022 JP 2022061122
(71) Applicant: GC Corporation, Sunto-gun, Shizuoka 410-1307 (JP)
(72) Inventor: UENO, Takayuki, Tokyo 174-8585 (JP); TAKADA, Daisuke, Tokyo 174-8585 (JP)
(74) Representative: Müller-Boré & Partner Patentanwälte PartG mbB
(86) International application number: PCT/JP2023/006074
(87) International publication number: WO 2023/189009

(57) **Abstract**

A method for producing a treated silica-dispersed monomer composition includes: mixing a basic silica dispersion obtained by dispersing silica in a solvent containing 10% by mass or greater of water with a silane coupling agent to prepare a silane-treated silica dispersion; mixing the silane-treated silica dispersion with a polymerizable monomer to obtain a mixture; and removing an aqueous phase from the mixture to obtain a composition in which the silica treated with the silane coupling agent is dispersed in the polymerizable monomer.

## Description

### TECHNICAL FIELD

The present invention relates to a method for producing a treated silica-dispersed monomer composition.

### BACKGROUND ART

In the field of dentistry, a composition in which silane-treated silica particles are dispersed in a polymerizable monomer is known (see, for example, PTLs 1 and 2). The composition is suitably applied to dental materials because it has good mechanical properties and a moderate fluidity (e.g., consistency).

### CITATION LIST

### PATENT LITERATURE

PTL 1: Japanese Patent No. 4748938
PTL 2: Japanese Patent No. 4800535

### SUMMARY OF THE INVENTION

### TECHNICAL PROBLEM

In the above-mentioned composition in which silane-treated silica particles are dispersed in a polymerizable monomer, the better the dispersion state of the silica particles in the polymerizable monomer, that is, the more uniformly the silica particles are dispersed by the polymerizable monomer, the better the dispersion state of the silica particles in a material when applied to the material and the better the properties of the material. However, there is room for improvement in the dispersion state of the silica particles in the composition obtained by the existing production method.

In view of the foregoing, an object of one embodiment of the present invention is to provide a method for producing a composition in which silane-treated silica particles are dispersed in a polymerizable monomer, by which a better dispersion state of the silica particles can be obtained.

### SOLUTION TO THE PROBLEM

A method for producing a treated silica-dispersed monomer composition according to an embodiment of the present invention includes mixing a basic silica dispersion obtained by dispersing silica in a solvent containing 10% by mass or greater of water with a silane coupling agent to prepare a silane-treated silica dispersion, mixing the silane-treated silica dispersion with a polymerizable monomer to obtain a mixture, and removing an aqueous phase from the mixture to obtain a composition in which the silica treated with the silane coupling agent is dispersed in the polymerizable monomer.

### ADVANTAGEOUS EFFECTS OF THE INVENTION

According to one embodiment of the present invention, a method for producing a composition in which silane-treated silica particles are dispersed in a polymerizable monomer can obtain a better dispersion state of the silica particles.

### DETAILED DESCRIPTION OF THE INVENTION

An embodiment of the present invention is a method for producing a composition (hereinafter referred to as a treated silica-dispersed monomer composition) in which silane-treated silica particles are dispersed in a polymerizable monomer. The production method according to the present embodiment includes at least a first step of mixing a silica dispersion obtained by dispersing silica in a solvent containing water with a silane coupling agent to prepare a silane-treated silica dispersion (may simply be referred to as a treated silica dispersion), a second step of adding a polymerizable monomer to the silane-treated silica dispersion to obtain a mixture, and a third step of removing an aqueous phase from the mixture to obtain a composition (silane-treated silica-dispersed monomer composition or treated silica-dispersed monomer composition) in which the silica treated with the silane coupling agent is dispersed in the polymerizable monomer.

### <First step: Preparation of the silane-treated silica dispersion>

The first step is a step of treating the surface of silica with a silane coupling agent, i.e., a step of reacting silane with a silane coupling agent. In this step, a basic silica dispersion obtained by dispersing silica in a solvent containing water is used. That is, a silane coupling agent is reacted on silica already dispersed in a dispersion, instead of bringing a silica coupling agent into direct contact with silica. As a result, since the silica and the silane coupling agent can react in the dispersion, it is possible to inhibit aggregation of the silica even if the silica has a small particle diameter, and to improve the dispersion state of the silica in a treated silica-dispersed monomer composition obtained by the production method of the present embodiment. That is, it is possible to inhibit aggregation of the silica and obtain a state in which the silica is uniformly dispersed in the composition.

The solvent of the silica dispersion contains water, and the amount of water is 10% by mass or greater relative to the total amount of the solvent of the silica dispersion. By using the above amount of water in the silica dispersion, it is possible to improve the dispersion state of the silica in the treated silica dispersion obtained in the first step, and in turn, to improve the dispersion state of the silica in the treated silica-dispersed monomer composition.

The amount of water in the solvent used in the silica dispersion may preferably be 20% by mass or greater, more preferably 30% by mass or greater, more preferably 50% by mass or greater, yet more preferably 70% by mass or greater, still more preferably 90% by mass or greater, and further more preferably 95% by mass or greater relative to the total amount of the solvent. It is also preferable that the solvent is substantially composed of water or is water.

In addition, the silica dispersion (the liquid before addition of either or both of the silane coupling agent and the monomer) may contain a solvent other than water. The solvent other than water may be an amphiphilic solvent (a solvent having both hydrophilic and hydrophobic properties) which can be mixed with water and has a lower boiling point than water, and specific examples include alcohols such as ethanol, methanol, propanol, isopropyl alcohol, butanol, and the like, acetones, ethers, and the like. When using the treated silica-dispersed monomer composition obtained according to the present embodiment for dental materials and the like, it is preferable to use ethanol from the viewpoint of safety in a case where the solvent remains.

Silica is added as an inorganic filler in the treated silica monomer composition obtained by the production method according to the present embodiment. The silica used in the present embodiment is nanoscale silica particles, i.e., particles that have an average primary particle diameter of less than 1 um. The average particle diameter of the silica is preferably from 1 nm through 500 nm, and more preferably from 1 nm through 200 nm. The silica particles having the above average particle diameter are preferred from the viewpoint of, for example, improving the aesthetic property of a dental material, when the treated silica-dispersed monomer composition produced is applied to the dental material. The silica particles may have one particle diameter peak or two or more particle diameter peaks in a measured particle diameter distribution of the silica. The particle diameter of the particles in the present specification may be measured by a laser diffraction scattering method or electron microscopy, and the average particle diameter may be the volume average diameter.

The silica used in the present embodiment may be non-aggregated silica. As used herein, non-aggregated silica has a spherical shape or a substantially spherical shape. Further, the BET specific surface area of the silica may be approximately from 20 m²/g through 500 m²/g.

The silica dispersion is basic. Therefore, the silica and the silane coupling agent can be reacted under a basic condition. The reaction under a basic condition is preferable because it is possible to promote the silica surface coupling reaction while inhibiting aggregation of the silica particles, and to improve the dispersion state of the silica in the treated silica-dispersed monomer composition to be obtained. The pH of the silica dispersion may be preferably from 8 through 12, and more preferably from 9 through 11.

The silica dispersion may be a silica sol, colloidal silica, or the like in which silica is dispersed in the solvent containing water. Examples of the silica dispersion include Ludox (registered trademark) PW-50 (pH of from 9.8 through 10.6), CL-X, and PX-30 available from W.R. GRACE and Company, Snowtex (registered trademark) ST-ZL (pH of from 9.0 through 11.0), ST-YL, and ST-30L available form Nissan Chemical Corporation, and the like. It is also possible to prepare the silica dispersion by dispersing dry silica particles such as fumed silica in the solvent containing water. In this case, examples of the dry silica particles include AEROSIL (registered trademark) OX-50, AEROSIL (registered trademark) 200, and AEROSIL (registered trademark) 380 available from EVONIK Industries AG, and the like.

A basic salt may be added to the solvent for preparing the silica dispersion, such that a basic aqueous solution may be prepared. The basic salt may be an inorganic salt or an organic salt, and may be, for example, sodium hydroxide, sodium tripolyphosphate, sodium carbonate, and the like. By making the silica dispersion basic by adding the basic salt, it is possible to reduce the possibility that the basicity of the silica dispersion may change due to volatilization or the like caused by temperature change, and to maintain the pH during the treatment process stably in the basic range. In addition, by sodium ions being contained in the silica dispersion, and by sodium ions being consequently contained in a mixture liquid of the silica dispersion and the silane coupling agent, it is possible to further inhibit aggregation of the silica particles in the liquid.

The concentration of the silica in the silica dispersion is preferably from 20% by mass through 70% by mass, more preferably from 30% by mass through 60% by mass. By setting the concentration in the above range, it is possible to inhibit aggregation of the silica and to increase the efficiency of the reaction between the silica and the silane coupling agent.

In the first step, the silica dispersion and the silane coupling agent are mixed. When doing so, the silane coupling agent may be added directly to the silica dispersion in an untreated state or may be added after at least a part of its hydrolyzable group is hydrolyzed. The use of the previously hydrolyzed silane coupling agent is preferable from the viewpoint that the surface treatment of the silica becomes quicker and the amount of a flammable solvent used can be reduced.

When previously hydrolyzing the silane coupling agent, the silane coupling agent is dispersed in an aqueous solvent such as water, an alcohol, or the like. In this case, the pH of the solvent in which the silane coupling agent is dispersed may be an acidic level or a basic level, yet it is preferable that the solvent in which the silane coupling agent is dispersed is neutral range or basic since it is preferable to promote the reaction between the silica and the silane coupling agent under a basic condition as described above. In addition, an acid component or a basic component other than the aqueous solvent may be added to the liquid in which the silane coupling agent is dispersed, in order to promote a hydrolysis reaction. Here, it is preferable that the mixture liquid obtained through mixing the liquid in which the silane coupling agent is dispersed with the silica dispersion is basic. When mixing the silane coupling agent with the silica dispersion after hydrolyzing the silane coupling agent, the silane coupling agent may be added in the form of a dispersion having a concentration of approximately from 20% by mass through 50% by mass.

The silane coupling agent is not particularly limited, but preferably has a polymerizable double bond, more specifically, a radical-polymerizable unsaturated double bond. The silane coupling agent having a radical-polymerizable unsaturated double bond is preferably a silane coupling agent having a (meth)acryloyloxy group, which is suitable in the dental field from the viewpoint of transparency. Specific examples of silane coupling agents having (meth)acryloyloxy groups include methacryloyloxymethyltrimethoxysilane, 2-methacryloyloxyethyltrimethoxysilane, 3-methacryloyloxypropyltrimethoxysilane (γ-methacryloxypropyltrimethoxysilane), 4-methacryloyloxybutyltrimethoxysilane, 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane (θ-methacryloxyoctyltrimethoxysilane), 9-methacryloyloxynonyltrimethoxysilane, 10-methacryloyloxydecyltrimethoxysilane, 11-methacryloyloxyundecyltrimethoxysilane, 12-methacryloyloxydodecyltrimethoxysilane, 13-methacryloyloxytridecyltrimethoxysilane, 11-methacryloyloxyundecyldichloromethylsilane, 11-methacryloyloxyundecyltrichlorosilane, 12-methacryloyloxydodecyldimethoxymethylsilane, 3-(meth)acryloyloxypropylmethyldimethoxysilane, 3-(meth)acryloyloxypropyltriethoxysilane, 3-(meth)acryloyloxypropylethyldiethoxysilane, 3-(meth)acryloyloxypropylmethyldiethoxysilane, 2-(meth)acryloyloxyethoxypropyltrimethoxysilane, and the like. Of these, one or more selected from 3-methacryloyloxypropyltrimethoxysilane (γ-methacryloxypropyltrimethoxysilane) and 8-methacryloyloxyoctyltrimethoxysilane (θ-methacryloxyoctyltrimethoxysilane) are preferable because they are commonly distributed and easily available. In addition, one or more of the above silane coupling agents may be used in combination.

When mixing the silane coupling agent with the silica dispersion, the required amount of the silane coupling agent or the dispersion of the silane coupling agent may be added at once, or the silane coupling agent or the dispersion of the silane coupling agent may be added dropwise in small amounts while the silica dispersion is stirred. When stirring the silica dispersion and the silane coupling agent, a stirring device such as a magnetic stirrer or the like may be used, or ultrasonic irradiation or the like may be performed.

### <Second step: Addition of polymerizable monomer>

The second step is a step of further mixing the silane-treated silica dispersion (treated silica dispersion) obtained in the first step with the polymerizable monomer to obtain a mixture. In the present embodiment, since the treated silica is dispersed in the liquid, stirring is easy even when the monomer is added. Compared with an integral blend method or the like in which silica is mixed with a monomer in advance, a state in which the silica is uniformly dispersed in the mixture is maintained.

A polymerizable monomer is a polymerizable double bond-containing monomer, more specifically, a monomer containing a radical-polymerizable unsaturated double bond. The polymerizable double bond-containing monomer is not particularly limited, yet is preferably a polymerizable monomer containing a (meth)acryloyloxy group suitable in the dental field from the viewpoint of a high transparency. The term "containing a (meth)acryloyloxy group" herein refers to having an acryloyloxy group, a methacryloyl group, or both. The term "(meth)acrylate" refers to a monomer, an oligomer, or a prepolymer of either or both of acrylate and methacrylate.

Examples of polymerizable monomers containing a (meth)acryloyloxy group include methyl (meth)acrylate, ethyl (meth)acrylate, isopropyl (meth)acrylate, n-butyl (meth)acrylate, isobutyl (meth)acrylate, hydroxypropyl (meth)acrylate, tetrahydrofurfuryl (meth)acrylate, glycidyl (meth)acrylate, 2-hydroxyethyl (meth)acrylate, 2-hydroxypropyl (meth)acrylate, 3-hydroxypropyl (meth)acrylate, 2-methoxyethyl (meth)acrylate, 2-ethoxyethyl (meth)acrylate, 2-ethylhexyl (meth)acrylate, benzyl (meth)acrylate, 2-hydroxy -1,3-di(meth)acryloxypropane, ethylene glycol di(meth)acrylate, diethylene glycol di(meth)acrylate, triethylene glycol di(meth)acrylate, butylene glycol di(meth)acrylate, neopentyl glycol di(meth)acrylate, 1,3-butanediol di(meth)acrylate, 1,4-butanediol di(meth)acrylate, 1,6-hexanediol di(meth)acrylate, trimethylolpropane tri(meth)acrylate, trimethylolethane tri(meth)acrylate, pentaerythritol tri(meth)acrylate, trimethylolmethane tri(meth)acrylate, pentaerythritol tetra(meth)acrylate, polybutylene glycol di(meth)acrylate, bisphenol A diglycidyl (meth)acrylate, and bismethacrylic acid [(dimethylmethylene) bis(4,1-phenyleneoxyethylene)] ester (Bis-MEPP). Further, specific examples of polymerizable monomers containing a (meth)acryloyloxy group include (meth)acrylates containing a urethane bond, such as di-2-(meth)acryloxyethyl-2,2,4-trimethylhexamethylenedicarbamate, 1,3,5-tris[1,3-bis{(meth)acryloyloxy}-2-propoxycarbonylaminohexane]-1,3,5-(1H,3H,5H)triazine-2,4,6-trione, 2,2-bis[4-{3-(meth)acryloyloxy-2-hydroxypropyl}phenyl]propane, 2,2-bis[4-{(meth)acryloyloxy}phenyl]propane, and the like. Further examples include (meth)acrylates of urethane oligomers containing 2,2'-di(4-hydroxycyclohexyl)propane, 2-oxypanone, hexamethylene diisocyanate, and 2-hydroxyethyl (meth)acrylate, meth acrylates of urethane oligomers containing 1,3-butanediol, hexamethylene diisocyanate, and 2-hydroxyethyl (meth)acrylate, and the like, and urethane dimethacrylate (UDMA). Among them, polyfunctional (meth)acrylates, especially di(meth)acrylates are preferable, and bismethacrylic acid [(dimethylmethylene)bis (4,1-phenyleneoxyethylene)] ester (Bis-MEPP) and urethane dimethacrylate (UDMA) are preferable. Polyfunctional (meth)acrylates are preferable since they have a higher boiling point than monofunctional (meth)acrylates and are less prone to volatilization and boiling in the subsequent heating step at from 80°C through 150°C (described below in a fourth step). The polymerizable monomers described above may be used alone or in combination of two or more kinds.

The amount of the polymerizable monomer and the amount of the silica may be adjusted in accordance with the characteristics of the treated silica-dispersed monomer composition to be obtained or a cured product thereof. Yet, the amount of the silica relative to 100 parts by mass of the polymerizable monomers may be preferably from 50 parts by mass through 300 parts by mass, and more preferably from 100 parts by mass through 250 parts by mass.

When mixing the treated silica dispersion obtained in the first step with the polymerizable monomer in the second step, the mixing may be performed using a mixer such as a dual-axis mixer.

### <Third step: Removal of the aqueous phase>

In this step, unnecessary components are removed from the mixture of the treated silica dispersion and the polymerizable monomer obtained in the second step described above, to extract the treated silica-dispersed monomer composition. The mixture at the end of the second step contains an oil phase and an aqueous phase. The oil phase mainly contains the polymerizable monomer, the silane-treated silica, and the residual silane coupling agents. On the other hand, the aqueous phase mainly contains water, the aqueous solvent, ions such as sodium ions, silica remaining untreated with silane, and the like.

In a case where the mixture at the end of the second step is visually clearly separated into an oil phase and an aqueous phase, it is possible to extract the oil phase of the supernatant by a publicly-known method such as a gradient method to remove the aqueous phase. In addition, regardless of whether the mixture at the end of the second step is clearly separated into an oil phase and an aqueous phase or not, either or both of separation of the mixture into the oil phase and the aqueous phase, and removal of the aqueous phase are possible by application of a certain energy such as a mechanical energy, a thermal energy, or the like to the mixture, as needed. The above treatment may be, for example, centrifugation, filtration, heating, freeze-drying, solvent evaporation, and the like. In a case of performing centrifugation for the separation into the oil phase and the aqueous phase, the specific gravity of the polymerizable monomer is preferably 1 or greater. Particularly, in a case where the separation into the oil phase and the aqueous phase cannot be observed visually, the mixture may be irradiated with ultrasonic waves so as to be separated into the oil phase and the aqueous phase.

Further, in the removal of the aqueous phase in the third step, water or acidic water may be added to the oil phase that is once extracted as described above, to disperse unnecessary components (for example, residual unreacted silane coupling agent, residual untreated silica, residual ions such as sodium ions, and the like) of the oil phase into an aqueous phase and remove the aqueous phase. Such addition of water or acidic water may also be performed to the mixture obtained at the end of the second step.

The oil phase left after the removal of the aqueous phase in the third step is the treated silica-dispersed monomer composition. The aqueous phase does not need to have been completely removed in the third step, and the aqueous phase may remain in the obtained treated silica-dispersed monomer composition (oil phase). However, it is preferable that no phase separation is observed in the treated silica-dispersed monomer composition. The state of the treated silica-dispersed monomer composition (oil phase) may be slurry, paste, or clay, provided that the treated silica-dispersed monomer composition has a certain degree of fluidity.

In this way, a composition (treated silica-dispersed monomer composition) in which the silica treated with the silane coupling agent is dispersed in the polymerizable monomer is obtained by the production method including at least the first to third steps described above. The dispersion state of the silica (filler) in the treated silica-dispersed monomer composition can be evaluated by direct observation of the composition, or observation of a cured product thereof obtained by curing the treated silica-dispersed monomer composition since a good dispersion state of the treated silica-dispersed monomer composition is reflected as a good dispersion state in the cured product. In this case, for example, evaluation can be performed by observation of aggregates in a flat surface or a cross-section of the cured product using an electron microscope or the like.

### <Fourth step: Heating>

The fourth step is a step of heating the oil phase obtained in the third step as needed. By heating, dehydration condensation of the silane coupling agent proceeds. Dehydration condensation of the silane coupling agent immobilizes the silane coupling agent on the silica surface and improves the dispersibility of the silica in the composition. In addition, by heating in the fourth step, the solvent can be removed through evaporation if any solvent has remained.

The heating temperature may be preferably from 80°C through 150°C, and more preferably from 100°C through 130°C. The heating time is preferably approximately from 30 minutes through 360 minutes.

### [Uses of the treated silica-dispersed monomer composition]

The obtained treated silica-dispersed monomer composition can be used for dental curable materials, for example, photopolymerizable dental curable materials. In particular, it can be suitably used for dental composite resins and one-pack dental composite resins. When used as dental composite resins, additional components, such as photopolymerization initiators, fillers such as barium glass, polymerization inhibitors, ultraviolet absorbers, fluorescent agents, pigments, and the like may be added to the above treated silica-dispersed monomer composition. Therefore, an embodiment of the present invention may be a method for producing a dental curable material by adding additional components to the above treated silica-dispersed monomer composition.

### Examples

In the following examples, compositions in which treated silica was dispersed in a monomer (treated silica monomer composition) were obtained by different methods. Cured products of the obtained compositions were prepared, and the dispersion state of the silica in the cured products was evaluated.

### <Silica dispersibility evaluation method>

A photopolymerizable composition was obtained by mixing a photopolymerization initiator (triethylene glycol dimethacrylate (TEGDMA) in which camphorquinone/dimethylaminoethyl methacrylate was dissolved) with the composition obtained in each of the aforementioned examples at a ratio of [the amount of the monomer in the composition] to [the amount of the TEGDMA] of 80:20. The photopolymerizable composition was polymerized by irradiation with visible light to produce a cured product, the surface of the cured product was mirror-polished with water-resistant polishing paper to form a glossy and smooth surface, and the glossy and smooth surface was observed using a Scanning Electron Microscope (SEM). Specifically, images of the glossy and smooth surface were captured at desirable three positions of the glossy and smooth surface at a magnification that would enable confirmation of a range of 50 µm × 40 µm. The number of aggregates equal to or larger than 3 µm was counted in each of the three images, and the average number of aggregates per image was calculated. The evaluation criteria were as follows.
Excellent: The average was less than 1.
Good: The average was 1 or greater and less than 3.
Fail: The average was 3 or greater.

### (Example 1)

Twenty grams of a colloidal silica aqueous dispersion ("Ludox (registered trademark) PW-50"obtained from W.R. GRACE and Company, having a silica concentration of 50% by mass, sodium ion-stabilized, having pH of from 9.8 through 10.6, and having silica particle diameter peaks at around from 20 nm through 30 nm and 50 nm) and 3.5 g of γ-methacryloxypropyltrimethoxysilane ("KBM -503" obtained from Shin-Etsu Chemical Co., Ltd.) were stirred in an environment of 23°C for 10 minutes using a magnetic stirrer, and then mixed with ultrasonic irradiation at an oscillation frequency of 38 kHz and at 120 W for 30 minutes, to obtain a treated silica dispersion.

Next, with addition of 5.5 g of bismethacrylic acid [(dimethylmethylene) bis(4,1-phenyleneoxyethylene)] (Bis-MEPP, a dimethacrylate monomer), the resulting product was further stirred using a dual-axis mixer at a revolution rate of 1,000 rpm for 10 minutes, to obtain a mixture.

Then, an aqueous phase was removed from the mixture, which had been separated into an oil phase and the aqueous phase, to obtain the oil phase. The obtained oil phase was further stirred with addition of 25 mL of acidic water (acetic acid aqueous solution having pH 3.5), to remove any separated aqueous phase. The resulting product was heated using a steam dryer at 120°C for 120 minutes, to obtain a treated silica-dispersed monomer composition (a composition in which the silica treated with γ-methacryloxypropyltrimethoxysilane was dispersed in the Bis-MEPP). A cured product was prepared using the obtained treated silica-dispersed monomer composition, and the dispersion state of the silica was evaluated as described above. The result was excellent.

### (Example 2)

A treated silica-dispersed monomer composition (a composition in which silica treated with γ-methacryloxypropyltrimethoxysilane was dispersed in Bis-MEPP) was obtained in the same manner as in Example 1, except that 20 g of another colloidal silica aqueous dispersion ("Snowtex (registered trademark) ST-ZL" obtained from Nissan Chemical Corporation, having a silica concentration of 40% by mass, sodium ion-stabilized, having pH of from 9.0 through 11.0, and having a silica average particle diameter of from 70 nm through 100 nm) was used instead of the colloidal silica aqueous dispersion of Example 1, and the amount of γ-methacryloxypropyltrimethoxysilane ("KBM -503" obtained from Shin-Etsu Chemical Co., Ltd.) was changed to 2.4 g and the amount of Bis-MEPP was changed to 5.0 g. A cured product was prepared using the obtained treated silica-dispersed monomer composition, and the dispersion state of the silica was evaluated as described above. The result was good.

### (Example 3)

Ten grams of fumed silica ("AEROSIL (registered trademark) OX-50" obtained from EVONIK Industries AG, having a silica average particle diameter of approximately 40 nm and a particle diameter distribution of from 10 nm through 100 nm) was added to 20 g of a sodium hydroxide aqueous solution adjusted to pH of 10, and mixed by stirring using a magnetic stirrer in an environment at 23°C for 10 minutes. Furthermore, the silica was dispersed to approximately the primary particle level by ultrasonic irradiation at an oscillation frequency of 38 kHz and at 120 W for 30 minutes, to obtain an aqueous silica dispersion (silica slurry). With addition of 4.0 g of γ-methacryloxypropyltrimethoxysilane ("KBM -503" obtained from Shin-Etsu Chemical Co., Ltd.), 30 g of the aqueous silica dispersion obtained was stirred using a magnetic stirrer in an environment at 23°C for 10 minutes, and further mixed with ultrasonic irradiation at an oscillation frequency of 38 kHz and at 120 W for 30 minutes, to obtain a treated silica dispersion.

A mixture was then obtained in the same manner as in Example 1 except that the amount of the Bis-MEPP to be added was changed to 6.0 g, and a treated silica-dispersed monomer composition (a composition in which silica treated with γ-methacryloxypropyltrimethoxysilane was dispersed in Bis-MEPP) was obtained. A cured product was prepared using the treated silica-dispersed monomer composition obtained, and the dispersion state of the silica was evaluated as described above. The result was good.

### (Example 4)

Twenty grams of a colloidal silica aqueous dispersion ("Ludox PW-50" obtained from W.R. GRACE and Company) was prepared. In the meantime, 3.0 g of θ-methacryloxyoctyltrimethoxysilane ("KBM -5803" obtained from Shin-Etsu Chemical Co., Ltd.) was dissolved in a mixed solvent of 40 g of ethanol and 10 g of distilled water, and stirred at room temperature for 24 hours, to hydrolyze θ-methacryloxyoctyltrimethoxysilane. The colloidal silica aqueous dispersion was further mixed with the solution of the hydrolyzed θ-methacryloxyoctyltrimethoxysilane by stirring, to obtain a treated silica dispersion.

Then, 6.0 g of Bis-MEPP was added to the silica dispersion and stirred, to obtain a mixture.

Ethanol was removed from the obtained mixture by evaporation, to obtain a mixture separated into an oil phase and an aqueous phase. The oil phase was extracted from the mixture, acidic water was added to the obtained oil phase and stirred, and then any aqueous phase was removed. The resulting product was heated at 120°C for 120 minutes using a steam dryer, to obtain a treated silica-dispersed monomer composition (a composition in which silica treated with θ-methacryloxypropyltrimethoxysilane was dispersed in Bis-MEPP). A cured product was prepared using the obtained treated silica-dispersed monomer composition, and the dispersion state of the silica was evaluated as described above. The result was excellent.

### (Example 5)

Twenty grams of a colloidal silica aqueous dispersion ("Ludox PW-50" obtained from W.R. GRACE and Company) was prepared. In the meantime, 3.5 g of γ-methacryloxypropyltrimethoxysilane ("KBM -503" obtained from Shin-Etsu Chemical Co., Ltd.) was dissolved in a mixed solvent of 40 g of ethanol and 10 g of distilled water, and stirred at room temperature for 24 hours, to hydrolyze γ-methacryloxypropyltrimethoxysilane. The colloidal silica aqueous dispersion and the solution of the hydrolyzed γ-methacryloxypropyltrimethoxysilane were mixed by stirring, to obtain a treated silica dispersion.

Then, 5.5 g of urethane dimethacrylate (UDMA, a dimethacrylate monomer) was added to the silica dispersion and stirred, to obtain a mixture.

The obtained mixture was subjected to evaporation in the same manner as in Example 4, to extract an oil phase and obtain a treated silica-dispersed monomer composition (a composition in which silica treated with γ-methacryloxypropyltrimethoxysilane was dispersed in UDMA). A cured product was prepared using the obtained treated silica-dispersed monomer composition, and the dispersion state of the silica was evaluated as described above. The result was excellent.

### (Comparative Example 1)

Ten grams of fumed silica ("AEROSIL OX-50" obtained from EVONIK Industries AG) was prepared in a mortar, and stirred in the mortar with dropwise addition of 3.5 g of γ-methacryloxypropyltrimethoxysilane ("KBM -503" obtained from Shin-Etsu Chemical Co., Ltd.). After being stirred until almost no lumps of silica (filler) were seen by visual observation, the silica was heated at 120°C for 120 minutes using a steam dryer, to condense γ-methacryloxypropyltrimethoxysilane (silane coupling agent) on the silica surface and obtain silica treated with the silane coupling agent (silane-treated silica). Subsequently, 5.5 g of Bis-MEPP was added to the silica treated with the silane coupling agent (silane-treated silica) and mixed, to obtain a treated silica-dispersed monomer composition (a composition in which silica treated with γ-methacryloxypropyltrimethoxysilane was dispersed in Bis-MEPP). A cured product was prepared using the obtained treated silica-dispersed monomer composition, and the dispersion state of the silica was evaluated as described above. The result was fail.

### (Comparative Example 2)

A treated silica-dispersed monomer composition (a composition in which silica treated with θ-methacryloxyoctyltrimethoxysilane was dispersed in UDMA) was obtained in the same manner as in Comparative Example 1 except that 3.0 g of θ-methacryloxyoctyltrimethoxysilane ("KBM-5803" obtained from Shin-Etsu Chemical Co., Ltd.) was used instead of 3.5 g of γ-methacryloxypropyltrimethoxysilane ("KBM-503" obtained from Shin-Etsu Chemical Co., Ltd.), and the amount of Bis-MEPP was changed to 6.0 g. A cured product was prepared using the obtained treated silica-dispersed monomer composition, and the dispersion state of the silica was evaluated as described above. The result was fail.

### (Comparative Example 3)

A treated silica-dispersed monomer composition (a composition in which silica treated with γ-methacryloxypropyltrimethoxysilane was dispersed in Bis-MEPP) was obtained in the same manner as in Example 1 except that 40 g of a colloidal silica aqueous dispersion ("Snowtex (registered trademark) ST-OL" obtained from Nissan Chemical Corporation, having a silica concentration of 20% by mass, having a pH of from 2.0 through 4.0, and having an average particle diameter of from 40 nm through 50 nm) was used instead of 20 g of the colloidal silica aqueous dispersion of Example 1, and the amount of γ-methacryloxypropyltrimethoxysilane ("KBM -503" obtained from Shin-Etsu Chemical Co., Ltd.) was changed to 2.4 g. A cured product was prepared using the obtained treated silica-dispersed monomer composition, and the dispersion state of the silica was evaluated as described above. The result was fail.

### (Comparative Example 4)

A treated silica dispersion was obtained in the same manner as in Example 3 except that 20 g of ethanol was used instead of 20 g of a sodium hydroxide aqueous solution adjusted to pH of 10.

Six point zero grams of Bis-MEPP was then added and stirred, to obtain a mixture.

Ethanol was removed from the mixture by evaporation, and then the mixture was heated at 120°C for 120 minutes using a steam dryer, to obtain a treated silica-dispersed monomer composition (a composition in which silica treated with γ-methacryloxypropyltrimethoxysilane was dispersed in Bis-MEPP). A cured product was prepared using the obtained treated silica-dispersed monomer composition, and the dispersion state of the silica was evaluated as described above. The result was fail.

Thus, in Example 1 to 4 including a step of mixing a silica dispersion obtained by dispersing silica in a solvent containing water with a silane coupling agent and treating the silica under a basic condition, the dispersion state in the cured products prepared using treated silica-dispersed monomer compositions thus obtained was found to be good in all of these Examples. In particular, the result of Example 1, in which colloidal silica was used and the particle diameter of the silica was relatively small, was excellent, whereas in Comparative Examples 1 and 2, in which silica was mixed with a silane coupling agent without using a solvent, the dispersion state of the silica in the cured products prepared using the obtained treated silica-dispersed monomer compositions was inferior to the results of Example 1 to 4. In Comparative Example 3, in which the reaction was performed under a solvent acidic condition, and in Comparative Example 4, in which the medium was ethanol, the dispersion state of the silica in the produced photopolymerizable compositions was inferior to the results of Example 1 to 4.

Although embodiments of the present invention have been described above, the present invention is not limited to the specific embodiments, and various modifications and changes are applicable within the scope of the invention described in the claims.

The present application claims priority under Japanese Patent Application No. 2022-061122 filed March 31, 2022, and the entire contents of the Japanese patent application are incorporated herein by reference.

## Claims

1. A method for producing a treated silica-dispersed monomer composition, the method comprising:
mixing a basic silica dispersion obtained by dispersing silica in a solvent containing 10% by mass or greater of water with a silane coupling agent to prepare a silane-treated silica dispersion;
mixing the silane-treated silica dispersion with a polymerizable monomer to obtain a mixture; and
removing an aqueous phase from the mixture to obtain a composition in which the silica treated with the silane coupling agent is dispersed in the polymerizable monomer.

2. The method for producing a treated silica-dispersed monomer composition according to claim 1,
wherein the polymerizable monomer is a polymerizable monomer containing a (meth)acryloyloxy group.

3. The method for producing a treated silica-dispersed monomer composition according to claim 2,
wherein the polymerizable monomer containing a (meth)acryloyloxy group is di(meth)acrylate.

4. The method for producing a treated silica-dispersed monomer composition according to any one of claims 1 to 3,
wherein the silane coupling agent is a silane coupling agent containing a (meth)acryloyloxy group.

5. The method for producing a treated silica-dispersed monomer composition according to any one of claims 1 to 4,
wherein the silica dispersion has a pH of from 9 through 11.

6. The method for producing a treated silica-dispersed monomer composition according to any one of claims 1 to 5,
wherein the silica is colloidal silica.

7. The method for producing a treated silica-dispersed monomer composition according to any one of claims 1 to 6,
wherein the solvent contains a sodium ion.

8. The method for producing a treated silica-dispersed monomer composition according to any one of claims 1 to 7,
wherein the removing the aqueous phase from the mixture includes removing an aqueous phase that is separated through stirring of the mixture with addition of water or acidic water.

9. The method for producing a treated silica-dispersed monomer composition according to any one of claims 1 to 8, further comprising after the removing the aqueous phase:
heating at from 80° C through 150° C.
